# EUROPÄISCHE PATENTANMELDUNG

(11) **EP 1 172 121 A1**
(43) Veröffentlichungstag der Anmeldung: **16.01.2002**
(21) Anmeldenummer: 00114191.0
(22) Anmeldetag: 13.07.2000
(51) Int. Cl.: A61M 15/00

(54) **Aerosoltherapienetzwerk**

(71) Anmelder: PARI GmbH Spezialisten für effektive Inhalation, 82319 Starnberg (DE)
(72) Erfinder: Sommer, Erik, 10115 Berlin (DE)
(74) Vertreter: HOFFMANN - EITLE

(57) **Zusammenfassung**

Die Erfindung beschreibt ein Kommunikationssystem, über das Aerosoltherapiedaten von Patientenbereichen 20, 21 in einen Datenbankserver 3 übertragen werden und dort so gespeichert sind, dass Therapeuten, Wissenschaftler, Pharmaunternehmen und Leistungsträger 4, 5 auf diese Daten zugreifen können.

## Beschreibung

Bei der Erfindung handelt es sich um ein System und ein Verfahren zur Übertragung von Therapiedaten und deren Sammlung und Auswertung, wobei durch technische Mittel der Austausch von Therapiedaten zwischen den Patienten auf der einen Seite und den Therapeuten bzw. Wissenschaftlern auf der anderen Seite bewerkstelligt wird.

Der Einsatz von Aerosoltherapiegeräten, mit denen ein Medikament in Form eines Aerosols einem Patienten zur Einatmung dargeboten wird, ist seit langem bekannt und erprobt.

Die Erfindung hat zum Ziel, den Einsatz derartiger Aerosoltherapiegeräte zu verbessern.

Dazu werden auf der Seite der Patienten Kommunikationseinrichtungen eingesetzt, mit deren Hilfe Aerosoltherapiedaten über eine Kommunikationsverbindung an eine zentrale Datensammelstelle (Server) übermittelt und dort gespeichert werden. Mögliche Datenkommunikationseinrichtungen sind beispielsweise Personal Computer (PC), auch in der Ausbildung als Notebook, Laptop, o.ä., mobile oder ortsfeste Telefone oder spezielle Kommunikationsgeräte, die allein für den Zweck der Übertragung der Therapiedaten ausgebildet und gegebenenfalls auch in ein Aerosoltherapiegerät integriert sind. Sofern diese Integration der Kommunikationseinrichtung in das Aerosoltherapiegerät nicht vorgesehen ist und auch keine unmittelbare Verbindung zwischen dem Therapiegerät und der Kommunikationseinrichtung besteht, muss der Patient die Therapiedaten, wie z.B. die Dauer der Therapie, die Medikamentenmenge, die Effektivität der Therapie, und sonstige physiologische/therapierelevante Daten, die von Fall zu Fall von Interesse sind, wie Lungenfunktionsparameter (z.B. PEF, FEV1) oder den therapeutischen Fortschritt kennzeichnende Daten, in die Telekommunikationseinrichtung eingeben, beispielsweise mit Hilfe eines speziellen Programms, das für den Personal Computer entwickelt wurde. Bei der Nutzung eines mobilen oder ortsfesten Telefons muss dabei auf die vorhandenen Eingabemöglichkeiten Rücksicht genommen werden. Es besteht aber die Möglichkeit, dass das Therapiegerät mit einer Schnittstelle ausgestattet wird, die die Übertragung der relevanten Therapiedaten in die Kommunikationseinrichtung erlaubt. Beispielsweise kann eine serielle Schnittstelle vorgesehen sein, die mit dem Personal Computer verbunden wird. Eine weitere geeignete Schnittstelle nutzt Infrarotlicht zur Übertragung (IR-Interface). Für die Erfindung von Bedeutung ist die Übertragung der Therapiedaten mit Hilfe der Kommunikationseinrichtung zum zentralen Server. Dort findet die systematische Speicherung der Daten statt, nachdem über die entsprechend ausgestattete Kommunikationsstelle der Server die Therapiedaten von den Patienten empfangen hat.

Zugriff auf den Therapiedatenserver besitzen einerseits Therapeuten und andererseits Wissenschaftler, sowie Leistungsträger zur Therapiekostenerstattung (Krankenkassen). Die Therapeuten betreuen einen oder mehrere Patienten unmittelbar, um auf der Basis der übermittelten Therapiedaten Entscheidungen bezüglich der Fortsetzung der Aerosoltherapie zu treffen. So kann durch entsprechende Auswahl und Auswertung der Therapiedaten dem Therapeuten die Möglichkeit gegeben werden, über eine Erhöhung oder Verringerung der Dosierung des Medikaments, eine Verlängerung oder Verkürzung der Therapiedauer, eine gleichmäßigere Verteilung mehrerer Therapiesitzungen auf einen Tag usw. zu entscheiden. Insbesondere bei der Unterstützung von Langzeit-Aerosoltherapien ist diese erfindungsgemäße Möglichkeit der Datenkommunikation vorteilhaft, da der Patient die Therapiedaten entsprechend den Therapiesitzungen mit Hilfe der erfindungsgemäß vorgesehenen Kommunikationseinrichtungen an den Therapieserver übermittelt, unabhängig davon, ob der Therapeut diese Daten sofort weiter auswertet. Dementsprechend kann der Therapeut unabhängig von der Therapiesitzung des Patienten eine Beurteilung der bereits gespeicherten Therapiedaten vornehmen und gegebenenfalls auf den Patienten zugehen, um ihm Vorschläge und Anweisungen bezüglich der Aerosoltherapie zu übermitteln. Dazu kann ebenfalls das erfindungsgemäße System genutzt werden, indem der Therapeut über den Therapiedatenserver und die Telekommunikationseinrichtung beim Patienten Therapieanweisungen oder -vorschläge übermittelt.

Der Therapeut benötigt lediglich einen Zugriff auf den Therapiedatenserver, ohne dass er für dessen Einrichtung und Betrieb verantwortlich ist.

Ebenfalls Zugriff auf den Therapiedatenserver kann für Wissenschaftler, Wissenschaftlergruppen oder pharmazeutische Unternehmen ermöglicht werden, wobei regelmäßig die einen Patienten identifizierenden Daten nicht zugänglich sind, sondern lediglich Therapiedaten, sofern sie für eine wissenschaftliche Untersuchung von Interesse sind. Der Wissenschaftler ruft vom Therapiedatenserver die Daten ab und führt die von ihm gewollten Analysen/Untersuchungen durch. Das erfindungsgemäße System erlaubt ihm an den Therapiedatenserver die Ergebnisse seiner Untersuchungen zu übermitteln, die dann regelmäßig anderen Wissenschaftlern, aber auch den Therapeuten, jedoch in der Regel nicht den Patienten zugänglich sind. Auf diese Weise unterstützt das erfindungsgemäße System die Zusammenarbeit zwischen den Wissenschaftlern und pharmazeutischen Unternehmen einerseits und den Therapeuten andererseits, so dass auch ausserhalb klinischer Versuche Therapiedaten für die Verbesserung von Aerosoltherapien gewonnen werden können.

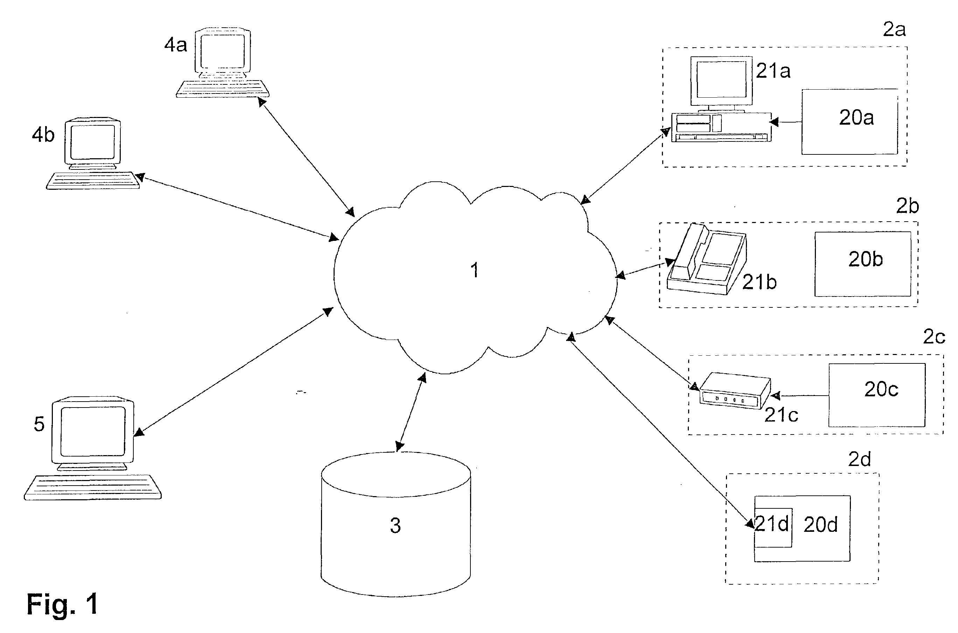

In Fig. 1 ist beispielhaft das erfindungsgemäße Datenübtertrgungssystem dargestellt.

Über ein Telekommunikationsnetz 1, beispielsweise das Telefonnetz oder das Internet, stehen Patientenbereiche 2a, 2b, 2c und 2d in Verbindung mit einem Therapiedatenbankserver 3. Von den Patientenbereichen 2a, 2b, 2c und 2d werden Aerosoltherapiedaten über das Kommunikationsnetz 1 an den Therapiedatenbankserver 3 übertragen, der diese Daten speichert und systematisch aufbereitet. Im Therapiedatenbankserver 3 ist auch die Verwaltung der Zugriffsrechte und Auswertemöglichkeiten implementiert.

In den Patientenbereichen 2a, 2b, 2c und 2d ist jeweils ein Aerosoltherapiegerät 20a, 20b, 20c, 20d vorhanden, mit dem der Patient die Aerosoltherapie durchführt. Ferner ist in jedem der Patientenbereiche 2a, 2b, 2c und 2d eine Telekommunikationseinrichtung 21a, 21b, 21c und 21d vorhanden, mit der eine Verbindung zum Telekommunikationsnetz 1 und zum Therapiedatenbankserver 3 aufgebaut werden kann.

Im Fall des Patientenbereichs 2a handelt es sich bei der Telekommunikationseinrichtung 21a um einen entsprechend ausgelegten Personal Computer (PC), der mit dem Aerosoltherapiegerät 20a über eine geeignete Schnittstelle in Verbindung steht, um von dem Aerosoltherapiegerät 20a Aerosoltherapiedaten zu empfangen. Der Personal Computer 21a übermittelt diese Daten dann über das Telekommunikationsnetz 1 an den Therapiedatenbankserver 3, wozu der Personal Computer 3 aus geeignete Weise mit dem Telekommunikationsnetz 1 verbunden ist. Der Patient kann den Personal Computer 21a dazu nutzen, weitere Therapiedaten einzugeben, die ebenfalls über das Telekommunikationsnetz 1 an den Therapiedatenbankserver 3 übermittelt werden. Die in Fig. 1 gezeigte direkte Verbindung zwischen Therapiegerät 20a und Personal Computer 21a ist nicht unbedingt erforderlich, da der Patient die auf diesem Wege übertragenen Daten ebenfalls eingeben kann, so dass der Personal Computer 21a die Therapiedaten dann nach der Eingabe durch den Patienten an den Therapiedatenbankserver weiterleitet.

Im Patientenbereich 2b ist ebenfalls ein Aerosoltherapiegerät 20b und eine Telekommunikationseinrichtung 21b vorgesehen. Bei der Telekommunikationseinrichtung des Patientenbereichs 2b handelt es sich um ein ortsfestes oder mobiles Telefon 21b, mit dem der Patient die Therapiedaten über das Telekommunikationsnetz 1 an den Therapiedatenbankserver 3 übermittelt. Dabei nutzt der Patient die Tastatur des ortsfesten oder mobilen Telefons 21b, um mit Hilfe der Zifferntasten, die in standardisierter Form auch mit Buchstaben bzw. Buchstabengruppen belegt sind oder frei belegt werden können, die Therapiedaten über das Telekommunikationsnetz 1 an den Therapiedatenbankserver 3 zu übertragen. Zumindest diejenigen Therapiedaten, die der Patient nicht selbst ermitteln kann, werden vom Aerosoltherapiegerät 20b angezeigt.

Im Patientenbereich 2c ist neben dem Aerosoltherapiegerät 20c eine Telekommunikationseinrichtung 21c in Form eines Modems vorgesehen, das unmittelbar mit dem Aerosoltherapiegerät 20c in Verbindung steht. Das Aerosoltherapiegerät 21c ist dafür ausgelegt, Therapiedaten über das Modem 21c und das Telekommunikationsnetz 1 an den Therapiedatenbankserver 3 zu übertragen.

Wie im Patientenbereich 2d gezeigt, kann die Telekommunikationseinrichtung 21d auch im Aerosoltherapiegerät 20d integriert sein.

Über das Telekommunikationsnetz 1 kann von Therapeuten, Wissenschaftlern und Pharmaunternehmen auf den Therapiedatenbankserver 3 und die dort abgelegten Daten zugegriffen werden.

Dazu sind bei den Therapeuten Zugriffseinrichtungen 4a und 4b, beispielsweise Personal Computer (PC) vorgesehen, die über das Telekommunikationsnetz 1 mit dem Therapiedatenbankserver 3 in Verbindung treten können. Die Zugriffseinrichtungen 4a und 4b erlauben einen Zugriff auf Therapiedaten spezieller Patienten, nämlich der Patienten, für die der Therapeut zuständig ist. Die Zugriffseinrichtungen 4a und 4b sind vorteilhaft universell ausgelegt, so dass verschiedene Therapeuten ein und dieselbe Zugriffseinrichtung 4a bzw. 4b nutzen können. Dabei wird die Identität des Therapeuten über Zugriffsmechanismen des Therapiedatenbankservers 3 gesteuert, beispielsweise durch Eingabe einer Benutzerkennung und eines Passwortes. Der Therapeut, der eine der Zugriffseinrichtungen 4a oder 4b nutzt, um auf die Therapiedaten seines Patienten zuzugreifen, erhält die Informationen, die für eine therapeutische Auswertung der Aerosoltherapiedaten erforderlich ist. Diese Informationen versetzen ihn in die Lage, den Fortgang der Aerosoltherapie seines Patienten zu überwachen und gegebenenfalls mit dem Patienten Kontakt aufzunehmen, um eine Änderung und/oder Anpassung der Aerosoltherapie zu vereinbaren.

Von der Zugriffseinrichtung 5, die für Wissenschaftler und Pharmaunternehmen vorgesehen ist, besteht ein Zugriff auf Aerosoltherapiedaten einer großen Patientengruppe, ohne jedoch die Möglichkeit der Individualisierung der Patientendaten. Das bedeutet, dass die Wissenschaftler oder Pharmaunternehmen, die über die Zugriffseinrichtung 5 auf den Therapiedatenbankserver 3 zugreifen, nicht in der Lage sind, Therapiedaten einem speziellen Patienten zuzuordnen. Jedoch erlaubt die Zugriffseinrichtung 6 einen Zugriff auf die Aerosoltherapiedaten des Therapiedatenbankservers 3 derart, dass beispielsweise statistische Auswertungen für ein spezielles Medikament oder eine spezielle Therapieform möglich sind.

Grundsätzlich regelt der Betreiber der Aerosoltherapiedatenbank 3 die Zugriffsmöglichkeiten für die Therapeuten 4a, 4b und die Wissenschaftler bzw. Pharmaunternehmen 6. Der Betreiber des Therapiedatenbankservers 3 kann auch vorsehen, dass Therapeuten und/oder Wissenschaftler bzw. Pharmaunternehmen Daten im Therapiedatenbankserver 3 ablegen können, die wiederum den anderen Nutzern, ggf. auch den Patienten, zur Verfügung stehen.

Der Betreiber der Therapiedatenbank 3 kann vorsehen, dass die Daten aufbereitet werden, bevor sie den Nutzern zur Verfügung gestellt werden. Beispielsweise können die Daten verdichtet werden, bevor Leistungsträger zur Therapiekostenerstattung auf die Daten zugreifen. Die verdichteten Daten können dann zur Therapiedatenkontrolle herangezogen werden.

## Patentansprüche

1. Aerosoltherapiedaten-Übertragungssystem mit
- einem Telekommunikationsnetz (1),
- Aerosoltherapiegeräten (20a, 20b, 20c, 20d), die in Patientenbereichen (2a, 2b, 2c, 2d) zur Nutzung durch Patienten im Rahmen von Aerosoltherapien aufgestellt sind,
- mit Telekommunikationseinrichtungen (21a, 21b, 21c, 21d), die in den Patientenbereichen (2a, 2b, 2c, 2d) vorgesehen sind und die mit dem Telekommunikationsnetz (1) zur Übertragung von Aerosoltherapiedaten verbunden sind,
- einem Aerosoltherapiedatenbankserver (3), der mit dem Telekommunikationsnetz (1) für die Übertragung und Speicherung der Aerosoltherapiedaten von den Patientenbereichen (2a, 2b, 2c, 2d) verbunden ist, und
- mit Zugriffseinrichtungen (4a, 4b, 5), die mit dem Telekommunikationsnetz (1) für den Zugriff auf den Therapiedatenbankserver (3) und die dort gespeicherten Aerosoltherapiedaten verbunden sind.

2. System nach Anspruch 1,
**dadurch gekennzeichnet, dass** die
Telekommunikationseinrichtung ein Personal Computer (21a) ist.

3. System nach Anspruch 1,
**dadurch gekennzeichnet, dass**
die Telekommunikationseinrichtung ein ortsfestes oder mobiles Telefon (21b) ist.

4. System nach Anspruch 1,
**dadurch gekennzeichnet, dass**
die Telekommunikationseinrichtung ein Modem (21c) ist, mit dem das Aerosoltherapiegerät (20c) verbunden ist.

5. System nach Anspruch 1,
**dadurch gekennzeichnet, dass**
die Telekommunikationseinrichtung (21d) in das Aerosoltherapiegerät (20d) integriert ist.

6. System nach einem der vorangegangenen Ansprüche,
**dadurch gekennzeichnet, dass**
die Zugriffseinrichtung (4a, 4b) für den Zugriff eines einen Patienten betreuenden Therapeuten ausgelegt ist.

7. System nach Anspruch 6,
**dadurch gekennzeichnet, dass**
die Zugriffseinrichtung (4a, 4b) des Therapeuten Zugriff auf Aerosoltherapiedaten und persönliche Daten eines Patienten gestattet, die im Therapiedatenbankserver (3) gespeichert sind.

8. System nach einem der vorangegangenen Ansprüche,
**dadurch gekennzeichnet, dass**
die Zugriffseinrichtung (6) für den Zugriff von Wissenschaftlern und Pharmaunternehmen ausgestaltet ist.

9. System nach Anspruch 8,
**dadurch gekennzeichnet, dass**
die Zugriffseinrichtung (5) für Wissenschaftler und Pharmaunternehmen keinen Zugriff auf die persönlichen Daten der Patienten gestattet.

10. Verfahren zur Übertragung und Speicherung von Aerosoldaten mit folgenden Schritten:
- Übertragen von Aerosoltherapiedaten mittels einer Telekommunikationseinrichtung (21a, 21b, 21c, 21d) von einem Patientenbereich (2a,2b, 2c, 2d) über ein Telekommunikationsnetz (1);
- Speicherung der übertragenen Aerosoltherapiedaten durch einen Aerosoltherapiedatenbankserver (3);
- Zugreifen auf die gespeicherten Aerosoltherapiedaten mittels Zugriffseinrichtungen (4a, 4b, 5) über das Telekommunikationsnetz (3).

11. Verfahren nach Anspruch 10, **gekennzeichnet durch** den Schritt:
- Aufbereiten der Aerosoltherapiedaten in dem Aerosoltherapiedatenbankserver (3).

12. Verfahren nach Anspruch 10 oder 11, **gekennzeichnet durch** den Schritt:
- Verdichten der Aerosoltherapiedaten in dem Aerosoltherapiedatenbankserver (3).
